# EUROPEAN PATENT SPECIFICATION

(11) **EP 2 800 585 B1**
(45) Date of publication and mention of the grant of the patent: **07.03.2018**
(21) Application number: 13700951.0
(22) Date of filing: 04.01.2013
(51) Int. Cl.: A61K 47/38, A61K 9/48, A23L 2/00

(54) **CELLULOSE ETHERS WITH IMPROVED THERMAL GEL STRENGTH**
CELLULOSEETHER MIT VERBESSERTER THERMISCHER GELSTÄRKE
ÉTHER CELLULOSIQUE AVEC UNE INTENSITÉ THERMIQUE DU GEL AMÉLIORÉE

(30) Priority: 06.01.2012 US 201261583790 P
(43) Date of publication of application: 12.11.2014
(73) Proprietor: Hercules Incorporated, Wilmington, DE 19808 (US)
(72) Inventor: BAKEEV, Kirill, Newark, DE 19702 (US); HUEBNER, Brian, John, Newark, DE 19702 (US); YANG, Hong, Newark, DE 19711 (US)
(74) Representative: Bülle, Jan
(86) International application number: PCT/US2013/020228
(87) International publication number: WO 2013/103771

(56) References cited:
- WO-A1-2013/009253
- US-A1- 2010 327 215
- ZIMMERMANN T. ET AL.: "Cellulose Fibrils for Polymer Reinforcement", ADVANCED ENGINEERING MATERIALS, vol. 6, no. 9, 2004, pages 754-761, XP002693417, Weinheim DOI: 10.1002/adem.200400097

## Description

### BACKGROUND OF THE INVENTION

### 1. Field of the Disclosed and Claimed Inventive Concepts

The presently disclosed and claimed inventive concept(s) relates generally to a thermal gelling composition having high-temperature gel strength. Specifically, the thermal gelling composition comprises water, water soluble thermal gelling cellulose ether and nanocrystalline cellulose (NCC), and has improved gel strength at temperatures above 25 °C when dispersed in water.

### 2. Background and Applicable Aspects of the Presently Disclosed and Claimed Inventive Concept(s)

Aqueous solutions of thermal gelling cellulose ethers demonstrate increased elastic or storage modulus (G') as defined by dynamic rheology measurements upon heating through a thermal gelation temperature. The thermal gelation temperature is the temperature at which a sharp rise in G' occurs. These polymers are used in a variety of different applications which take advantage of their high-temperature gel strength. They may be used as binders for food products to maintain consistency of the food products during heating. They can also be used as binding agents for inorganic products, in particular, unfired, or green ceramic bodies. Thermal gelling cellulose ethers can also be used to form capsules in pharmaceuticals.

Typically, aqueous solutions of thermal gelling cellulose ethers first demonstrate a decrease in elastic modulus when heated. The decrease in elastic modulus is then followed by a rapid increase in elastic modulus upon a further increase in temperature. The elastic modulus of the thermal gelling cellulose ether solution is the lowest at the temperature immediately before it begins to increase. As the elastic modulus decreases, the efficacy of the thermal gelling cellulose ether as a binder also decreases.

Modifying the gel strength and gel temperature of thermal gelling cellulose ethers is very difficult. It can be accomplished to a limited extent by adjusting the chemical composition and molecular weight, but this is very limited.

US 2010/037215 discloses an aircraft anti-icing composition including a freezing point depressant and a thickener including nanocrystalline cellulose. A thickening composition also includes nanocrystalline cellulose. Also taught is the use of nanocrystalline cellulose in the manufacture of anti-icing compositions and the use of nanocrystalline cellulose in a thickening composition.

ZIMMERMANN, T.; ET AL.: "Cellulose Fibrils for Polymer Reinforcement", ADVANCED ENGINEERING MATERIALS 2004; vol. 6, no. 9, pages 754-761, Weinheim Verlag; DOI:10.1002/adem.2004400097; relates to lignocellulosic fibres obtained from renewable resources such as wood as paint or ink thickener, pharmaceutical tablet binder or rheology-control agent in food.

### BRIEF DESCRIPTION OF DRAWINGS

FIG. 1 is a graph showing dynamic rheology data-elastic modulus (G') and viscous modulus (G") for 2 wt% aqueous dispersions of MHPC (methylhydroxypropyl cellulose) and MHPC-NCC blends, versus temperature.
FIG. 2 is a graph showing elastic modulus for 2 wt% aqueous dispersions of MHPC-NCC blends at 90 °C versus the NCC weight percent in the blend.
FIG. 3 is a graph showing elastic modulus for 2 wt% aqueous dispersions of MHEC (methylhydroxyethyl cellulose) -NCC dispersions at 90 °C versus the NCC weight percent in the blend.
FIG. 4 is a graph showing elastic modulus for 2 wt% aqueous dispersions of MC (methyl celluIose)-NCC blends at 50 °C versus the NCC weight percent in the blend.

### DETAILED DESCRIPTION

Before explaining at least one embodiment of the inventive concept(s) in detail by way of exemplary drawings, experimentation, results, and laboratory procedures, it is to be understood that the inventive concept(s) is not limited in its application to the details of construction and the arrangement of the components set forth in the following description or illustrated in the drawings, experimentation and/or results. The inventive concept(s) is capable of other embodiments or of being practiced or carried out in various ways. As such, the language used herein is intended to be given the broadest possible scope and meaning; and the embodiments are meant to be exemplary - not exhaustive. Also, it is to be understood that the phraseology and terminology employed herein is for the purpose of description and should not be regarded as limiting.

Unless otherwise defined herein, scientific and technical terms used in connection with the presently disclosed and claimed inventive concept(s) shall have the meanings that are commonly understood by those of ordinary skill in the art. Further, unless otherwise required by context, singular terms shall include pluralities and plural terms shall include the singular. Generally, nomenclatures utilized in connection with, and techniques of chemistry described herein are those well known and commonly used in the art. Reactions and purification techniques are performed according to manufacturer's specifications or as commonly accomplished in the art or as described herein. The nomenclatures utilized in connection with, and the laboratory procedures and techniques of, analytical chemistry, synthetic organic chemistry, and medicinal and pharmaceutical chemistry described herein are those well known and commonly used in the art. Standard techniques are used for chemical syntheses, chemical analysis, pharmaceutical preparation, formulation, and delivery, and treatment of patients.

All patents, published patent applications, and non-patent publications mentioned in the specification are indicative of the level of skill of those skilled in the art to which this presently disclosed and claimed inventive concept(s) pertains.

All of the compositions and/or methods disclosed and claimed herein can be made and executed without undue experimentation in light of the present disclosure. While the compositions and methods of this invention have been described in terms of preferred embodiments, it will be apparent to those of skill in the art that variations may be applied to the compositions and/or methods and in the steps or in the sequence of steps of the method described herein without departing from the concept, spirit and scope of the invention.

As utilized in accordance with the present disclosure, the following terms, unless otherwise indicated, shall be understood to have the following meanings:

The use of the word "a" or "an" when used in conjunction with the term "comprising" in the claims and/or the specification may mean "one," but it is also consistent with the meaning of "one or more," "at least one," and "one or more than one." The use of the term "or" in the claims is used to mean "and/or" unless explicitly indicated to refer to alternatives only or the alternatives are mutually exclusive, although the disclosure supports a definition that refers to only alternatives and "and/or." Throughout this application, the term "about" is used to indicate that a value includes the inherent variation of error for the device, the method being employed to determine the value, and/or the variation that exists among the study subjects. The use of the term "at least one" will be understood to include one as well as any quantity more than one, including but not limited to, 2, 3, 4, 5, 10, 15, 20, 30, 40, 50, 100, etc. The term "at least one" may extend up to 100 or 1000 or more, depending on the term to which it is attached; in addition, the quantities of 100/1000 are not to be considered limiting, as higher limits may also produce satisfactory results. In addition, the use of the term "at least one of X, Y and Z" will be understood to include X alone, Y alone, and Z alone, as well as any combination of X, Y and Z.

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

The term "or combinations thereof" as used herein refers to all permutations and combinations of the listed items preceding the term. For example, "A, B, C, or combinations thereof" is intended to include at least one of: A, B, C, AB, AC, BC, or ABC, and if order is important in a particular context, also BA, CA, CB, CBA, BCA, ACB, BAC, or CAB. Continuing with this example, expressly included are combinations that contain repeats of one or more item or term, such as BB, AAA, MB, BBC, AAABCCCC, CBBAAA, CABABB, and so forth. The skilled artisan will understand that typically there is no limit on the number of items or terms in any combination, unless otherwise apparent from the context.

The presently disclosed and claimed inventive concept(s) is premised on the discovery that one can improve the thermogelation behavior of thermal gelling cellulose ethers by combining nanocrystalline cellulose with thermal gelling cellulose ethers. This combination can provide an increase in gel strength of the nanocrystalline cellulose/thermal gelling cellulose ether blend versus the thermal gelling cellulose ether by itself. This increase in gel strength can be observed over a broad temperature range.

This combination is particularly beneficial for binder applications such as food products and advanced ceramics manufacturing, as well as use in the formation of capsules.

According to the presently disclosed and claimed inventive concept(s), aqueous dispersions of cellulose ethers with improved thermal gel strength comprise thermal gelling cellulose ether, nanocrystalline cellulose and water. As demonstrated in the examples, gel strength or apparent gel strength is measured as the storage modulus of the aqueous dispersion of the thermal gelling cellulose ether and nanocrystalline cellulose. The terms are used interchangeably herein.

Nanocrystalline cellulose is a crystalline portion of cellulose which can be formed by acid hydrolysis of cellulose combined with mechanical treatment. These nanometer size cellulose particles are crystalline in nature, insoluble in water, stable, chemically inactive and physiologically inert with attractive binding properties.

Cellulose is one of the most abundant biopolymers on earth, occurring in wood, cotton, hemp and other plant-based material and serving as the dominant reinforcing phase in plant structures. Cellulose can also be synthesized by algae, tunicates, and some bacteria. It is a homopolymer of glucose repeating units which are connected by 1-4 βglycosidic linkages. The 1-4 β-linkages form cellulose in linear chains, which interact strongly with each other through hydrogen bonds. Because of their regular structure and strong hydrogen bonds, cellulose polymers are highly crystalline and aggregate to form substructures and microfibrils. Microfibrils, in turn aggregate to form cellulosic fibers.

Purified cellulose from wood or agricultural biomass can be extensively disintegrated or produced by bacterial processes. If the cellulosic material is composed of nanosized fibers, and the properties of the material are determined by its nanofiber structure, these polymers are described as nanocelluloses or nanocrystalline cellulose. The terms are used interchangeably herein.

In general, nanocelluloses are rod shaped fibrils with a length/diameter ratio of approximately 20 to 200. In one non-limiting embodiment, the nanocelluloses have a diameter less than about 60 nm. In another non-limiting embodiment, the nanocelluloses have a diameter between about 4 nm to about 15 nm, and a length of about 150 nm to about 350 nm. The size and shape of the crystals vary with their origins. For example, but not by way of limitation, nanocrystalline cellulose from wood can be about 3 to about 5 nm in width and about 20 to about 200 nm in length. Other nanocrystalline cellulose obtained from other sources such as cotton may have slightly different dimensions.

The nanocrystalline cellulose has high stiffness, large specific surface area, high aspect ratio, low density and reactive surfaces that can facilitate chemical grafting and modification. At the same time, the material is inert to many organic and inorganic substances.

The production of nanocrystalline cellulose by fibrillation of cellulose fibers into nano-scale elements requires intensive mechanical treatment. However, depending upon the raw material and the degree of processing, chemical treatments may be applied prior to mechanical fibrillation. Generally preparation of nanocrystalline cellulose can be described by two methods, acid hydrolysis and mechanical defibrillation. In the first method, nanocellulose can be prepared from the chemical pulp of wood or agricultural fiber mainly by acid hydrolysis to remove the amorphous regions, which then produce nano-size fibrils. The hydrolysis conditions are known to affect the properties of the resulting nanocrystals. Different acids also affect the suspension properties. Nanocrystal size, dimensions, and shape can also be determined to a certain extent by the nature of the cellulose source.

The acid hydrolysis can be conducted using a strong acid under strictly controlled conditions of temperature, agitation and time. The nature of the acid and the acid-to-cellulosic ratio are also important parameters that affect the preparation of nanocellulose. Examples of the acids can include, but are not limited to, sulfuric acid, hydrochloric acid, phosphoric acid and hydrobromic acid. The hydrolysis temperature can range from room temperature up to about 70 °C and the corresponding hydrolysis time can be varied from about 30 minutes to about 12 hours depending on the temperatures. Immediately following hydrolysis, suspension can be diluted to stop the reaction.

In one non-limiting embodiment, the suspension can be diluted from about fivefold to about ten-fold to stop the reaction. Then the suspension can be centrifuged, washed once with water and re-centrifuged and washed again. This process can be repeated for about four to five times to reduce the acid content. Regenerated cellulose dialysis tubes or Spectrum Spectra/Pro regenerated cellulose dialysis membrane having a molecular cutoff of about 12,000-14,000 can be used to dialyze the suspension against distilled water for several days until the water pH reach a constant value, for example but not by way of limitation, a pH value of about 7.0.

To further disperse and reduce the size of the cellulose crystals, the suspensions of cellulose crystals can be processed by either sonicating or passing through a high shear micro fluidizer. This kind of prepared material is referred to as nanocellulose, nanocrystalline cellulose (NCC), cellulose nanocrystals, cellulose nanofibres or cellulose whiskers.

The second method is primarily a physical treatment. Bundles of microfibrils called cellulose microfibril or microfibrillated cellulose with diameters from tens of nanometers (nm) to micrometers (µm) are generated by using high pressure homogenizing and grinding treatments. A novel process using high-intensity ultrasonication has also been used to isolate fibrils from natural cellulose fibers. High intensity ultrasound can produce very strong mechanical oscillating power, so the separation of cellulose fibrils from biomass is possible by the action of hydrodynamic forces of ultrasound. This method can produce a microfibrillated cellulose with a diameter with a diameter less than about 60 nm. In one non-limiting embodiment, a microfibrillated cellulose between about 4 nm to about 15 nm, and a length less than 1000 nm. The microfibrillated cellulose can optionally further undergo chemical, enzymatic and/or mechanical treatment. Both methods for preparing nanocrystalline cellulose are described in U.S. Patent No. 8,105,430.

There are a variety of different thermal gelling cellulose ethers. The gellation temperature can be varied depending upon the particular composition. Any thermal gelling water soluble cellulose ether can be used in the presently disclosed and claimed inventive concept(s). Examples of the thermal gelling cellulose ethers typically employed as binders can include, but are not limited to, methyl cellulose (MC), methylhydroxypropyl cellulose (MHPC), methylhydroxyethyl cellulose (MHEC), and combinations of thereof.

The ratio of nanocrystalline cellulose to thermally gelling cellulose ether can be varied depending upon the particular application. Generally, the composition can include up to about 70% by weight of the nanocrystalline cellulose to as low as about 10% or less nanocrystalline cellulose. The ratios and their benefits are shown in more detail in the examples below, and the accompanying data. Generally, the ratio of thermally gelling cellulose ether to nanocrystalline cellulose can be varied from about 10:1 to about 1:2 by weight. The composition is substantially free of alkylene glycols or other freeze point suppressants.

The nanocrystalline cellulose can be blended with the thermal gelling cellulose in water or can be dry blended, depending on the intended use. The nanocrystalline cellulose disperses in water, and the cellulose ether can be dissolved in water at temperatures below its gelation temperature. Hereinafter this aqueous system is referred to as an aqueous dispersion and means that the thermal gelling cellulose ether is substantially dissolved at temperatures below its gelation temperature and the nanocrystalline cellulose is dispersed in that system. In one non-limiting embodiment, the aqueous dispersion of thermal gelling cellulose ether and nanocrystalline cellulose can include from about 0.1 to about 40 weight percent water soluble thermal gelling cellulose ether and from about 0.1 to about 10 percent by weight nanocrystalline cellulose with the remainder being water. In another non-limiting embodiment, the aqueous dispersion of thermal gelling cellulose ether and nanocrystalline cellulose can include from about 0.1 to about 10 weight percent water soluble thermal gelling cellulose ether and from about 0.1 to about 5 percent by weight nanocrystalline cellulose with the remainder being water.

In one non-limiting embodiment, the solution can include from about 0.1 wt% to about 20 wt% of the combined thermal gelling cellulose ether and nanocrystalline cellulose. These concentrations can be varied depending upon the particular application. When used as a binder for food or ceramic manufacture, binder solutions with lower concentrations of thermal gelling cellulose ether/NCC, generally around 0.4 wt% to about 2 wt%, can be used. Whereas with formation of capsule walls a higher concentration, generally 20 wt% or more, can be employed. When added to water containing products such as water containing foods, the binders can be added as a powder.

The aqueous dispersion can be formed by simply blending the dry components, the thermal gelling cellulose ether and the nanocrystalline cellulose, in water at room temperature. Alternately, the thermal gelling cellulose ether can be dry or wet blended with the nanocrystalline cellulose in the desired ratio. The dry blend can be combined with a product that has sufficient moisture to dissolve the thermal gelling cellulose ether and disperse the nanocrystalline cellulose.

The thermal gelling cellulose ether/nanocrystalline cellulose blend of the presently disclosed and claimed inventive concept(s) can be combined with non-thermogelling water soluble polymers, for example but by no way of limitation, hydroxyethyl cellulose, carboxymethyl cellulose as well as acrylates and others to effect their modulus, and in some applications, to reduce costs.

The following examples illustrate the presently disclosed and claimed inventive concept(s), parts and percentages being by weight, unless otherwise indicated, Each example is provided by way of explanation of the presently disclosed and claimed inventive concept(s), not limitation of the presently disclosed and claimed inventive concept(s).

### EXAMPLES

### Materials

Nanocrystalline cellulose (NCC) was prepared using the procedure similar to D. G. Gray et al, Langmuir, V.12, p. 2076-2082 (1996). The details of the procedure are given below.

Prior to acid hydrolysis, the pulp was fluffed using a grinder. The fluffed pulp was hydrolysed in about 65% of sulfuric acid at about 45 °C for about 2 hrs. The solid content of the reaction was about 10%. First, the fluffed pulp was added into acid in a water bath with mechanical mixing and allowed to hydrolyze. After hydrolysis, the cellulose suspension was diluted with deionized (DI) water (∼10 times volumes of the acid solution) and centrifuged twice to remove the acid. 2% sodium carbonate solution was then added to neutralize the residue acid. The suspension was dialyzed with DI water to remove the salt. The purified suspension was dried to determine the yield.

The following thermal gelling cellulose ethers were used, which are the commercial products of Ashland, Inc.
MHPC 1034R - Culminal® MHPC 1034R
MHEC 40000 - Culminal® MHEC 40000
MC A4M - Benecel® A4M
MHPC 734R- Culminal® MHPC 734R
MHEC C4053 - Culminal® MHEC C4053

Ethylene glycol was received from Mallfnckrodt Chemical, 99% purity and used as is.

### Formulations

All aqueous systems mentioned in the examples below were prepared by dissolving thermal gelling cellulose ether (CE) or dissolving/dispersing dry blend of CE/NCC in water using two major protocols:
1. Hot-cold method as described below.
   Hot DI water (∼ 90 °C) was added to CE or CE/NCC (moisture corrected) while mixing at about 1000 rpm for about 15 min.
   Ice bath around jar was placed to allow solution to cool (∼15 min) - reduce speed to about 700 rpm.
   Ice bath was removed and mixing was continued at about 700 rpm for about 30 min. The solution was placed in a constant temp bath and cooled to about 25 °C.

This protocol was used for CE and CE/NCC blends based on methylhydroxypropylcellulose (MHPC) and methylhydroxyethylcellulose (MHEC).
2. "Conventional" method as described below:
   Dry CE was dissolved and/or dry CE/NCC blend was dispersed in water with overhead mixing for about 2-3 hours at about 500 rpm.

This protocol was used for CE and CE/NCC blends based on methylcellulose (MC).

### Measurements

Molecular characterization of thermal gelling cellulose ethers including degree of substitution, molar substitution were conducted according to well-established industry protocols:
ASTM, D 4794, re-approved 2009.
Hodges et al., Anal. Chern., v. 51, N13, p. 2172 (1972)

Percent of un-substituted anhydroglucose units (un-sub. AGUs) was measured in cellulose ethers according to:
Richardson N.S. et al. Biomacromolecules, v 3, p. 1359-1363 (2002)
Richardson N.S. et al, Anal. Chern, v 75, p 6077-6085 (2003)

Rheological measurements for gel strength analysis were performed similarly to procedures described in:
US 6,235,893 B1 by Reibert et al.
Dapcevic-Hadnadev T. et al. in Food processing, Quality and safety, 3-4, p. 69-73 (2009).

According to the above references, elastic modulus (G') measured is also referred to as gel strength.

The procedure details:
AR-G2 stress controlled rheometer
Couette geometry
Gap 5920 micron
Peltier temperature control
Perform Dynamic Temperature Ramp dynamic test at 1Hz of frequency, 0.5% of Strain, and 1 °C/min from about 25 °C to about 92 °C.

Thermo gelation phenomenon is well known for a class of nonionic cellulose ether polymers such as MC, MHPC, MHEC and typically is referred to as an increase in G' or gel strength of the polymer in aqueous solution upon increase in temperature (T) within a defined T-range.

Some representative references on this topic include:
Desbrieres J. et al., Polymer, v. 41, p. 2451-2461 (2000).
Sarkar N., Carbohydrate polymers, v. 26, p. 195-203 (1995).
Sarkar N., Appl. J. Polym. Sci., v. 24, p. 1073-1087 (1979).

### Example 1

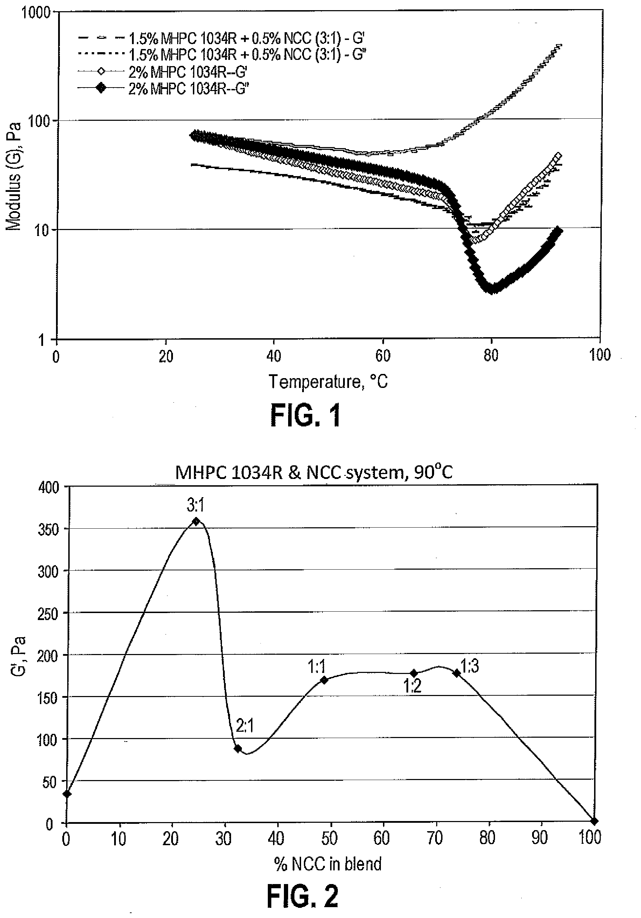

Dynamic rheology data for MHPC 1034R product (2% in water) and the 3:1 blend of MHPC 1034R to NCC respectively at 2% blend concentration in water are shown in Figure 1. Both elastic modulus (G') and viscous modulus (G") are shown for individual components - CE, NCC and their blend. In the case of NCC material, both G' and G" are in background noise, i.e. around zero. In the case of MHPC polymer there is a thermal gelation effect manifested in sharp G' upturn above approximately 80 °C.

The MHPC/NCC (3:1) blend shows a boost in G' or gel strength of the MHPC product starting at about 65 °C and above. This type of unexpected behavior is synergistic in nature as the effect is not typical of individual components - MHPC and NCC tested at the same total concentration as the blend.

### Example 2

Figure 2 captures the effect observed in Figure 1 but as a function of CE/NCC blend ratio at a constant T = 90 °C and constant blend concentration of 2 wt% in water. The synergy effect is even more pronounced (about 10 times gain in G') at lower concentrations of NCC in the blend, i.e. MHPC/NCC = 3:1 ratio. The effect is dependent on CE/NCC ratio in the blend.

### Example 3

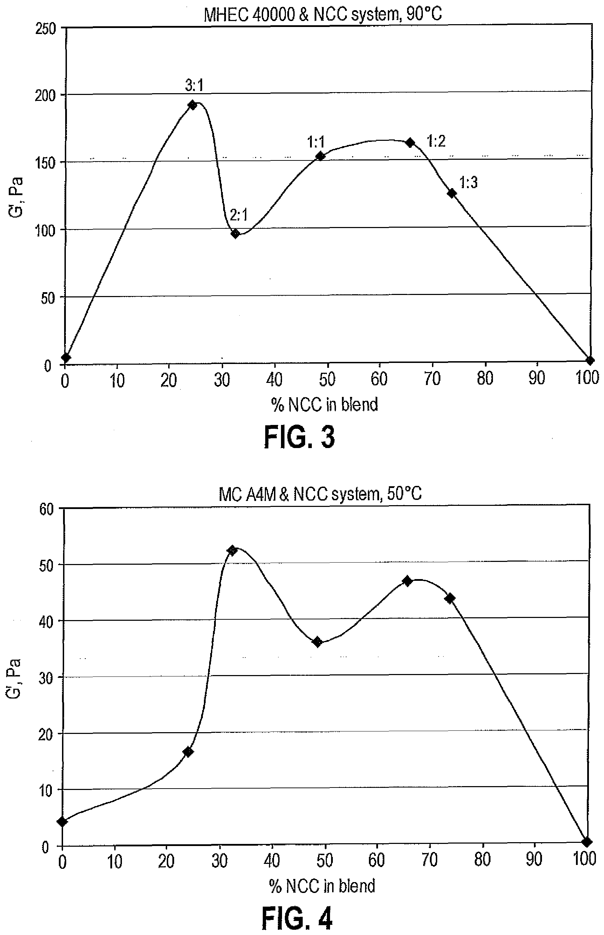

Figure 3 captures the relationship between G' and CE/NCC blend composition/ratio at a constant T = 90 °C and constant blend concentration of 2 wt% in water for MHEC polymer. The synergy effect is very strongly pronounced-more than 30 times gain in G' over MHPC control at lower concentrations of NCC in the blend, i.e. MHPC/NCC = 3:1 ratio. The effect is dependent on CE/NCC ratio in the blend.

The trend is similar, overall, to the data for MHPC polymer presented in Figure 2.

### Example 4

Figure 4 captures the relationship between G' and CE/NCC blend composition/ratio at a constant T = 50 °C and constant blend concentration of 2 wt% in water for MC polymer. The synergy effect is clearly pronounced - about 10 times gain in G' over MC control at lower concentrations of NCC in the blend, i.e. MC/NCC = 2:1 ratio. The effect is dependent on CE/NCC ratio in the blend.

The overall trend is similar to the data for MHPC & MHEC polymers presented above. However, the distinction is that the synergy is observed in this case at lower T of 50 °C, with no synergy observed at higher T of 90 °C.

### Example 5

This example captures G' data for CE and CE/NCC blend at a fixed CE/NCC ratio (2:1) and 2 wt% total product concentration in water phase. A range of CEs were tested including two MHPC products, two MHEC products, and one MC product. The results are given for various temperatures to capture major benefits for CE/NCC blend over CE alone depending on type of CE used.

There is a correlation between lower degree of methyl substitution (Me-DS) for a CE and G' synergy effect within a defined CE family. For example, at 90 °C, MHPC 1034R polymer has clear G' synergy with NCC at 2:1 ratio, while MHPC 734R does not, that correlates with lower Me-DS values and higher percent of un-substituted AGUs for MHPC 734R. Similar trend was observed for MHEC polymers, where MHEC 40000 had higher G' synergy, lower Me-DS and higher percent of un-substituted AGUs compared to MHEC C4053. Table 1 shows all these results.

**Table 1**

| System-Property | G'@50°C [Pa] | G'@65°C [Pa] | G'@90°C [Pa] | % unsub AGU | HE-MS | HP (wt%) | Me-DS |
|---|---|---|---|---|---|---|---|
| MHPC 1034R | | 22 | 34.2 | 8.36 | | 9.74 | 1.43 |
| NCC | | 0 | 0 | | | | |
| 1.33 % MHPC 1034R + 0.67% NCC | | 31 | 88.19 | | | | |
| MHPC 734 R | | 1.98 | 237.6 | 2.72 | | 7.06 | 1.81 |
| NCC | | 0 | 0 | | | | |
| 1.33 % MHEC 734R + 0.67% NCC | | 25.4 | 117.1 | | | | |
| MHPC 40000 | | | 4.97 | 9.67 | 0.25 | | 1.44 |
| NCC | | | 0 | | | | |
| 1.33 % MHEC 40000 + 0.67% NCC | | | 107.4 | | | | |
| MHPC C4053 | | | 34.74 | 6.78 | 0.27 | | 1.79 |
| NCC | | | 0 | | | | |
| 1.33 % MHEC C4053 + 0.67% NCC | | | 52.32 | | | | |
| MC A4M | 4.22 | | | 5.51 | | | 1.80 |
| NCC | 0 | | | | | | |
| 1.33 % MC A4M + 0.67% NCC | 52.2 | | | | | | |

| | | | | | | | |
|---|---|---|---|---|---|---|---|
| HE-MS - hydroxyethyl molar substitution HP - hydroxypropyl content Me-DS - methyl degree of substitution % un-substituted AGU - percent of un-substituted anhydroglucose units in cellulose ether. | | | | | | | |

The thermal gelling cellulose ether/nanocrystalline cellulose blend can be used in a variety of different applications, several of which are discussed below. The thermal gelling cellulose ether/nanocrystalline cellulose blend can be useful in a variety of food compositions as a binder. Examples of food compositions can include, but are not limited to, vegetables, meats, soy patties, reformed seafood, reformed cheese sticks, cream soups, gravies, sauces, salad dressing, mayonnaise, onion rings, jams, jellies, syrups, pie filling, potato products such as extruded fries, batters for fried foods, pancakes/waffles and cakes, pet foods, beverages, frozen desserts, cultured dairy products such as ice cream, cottage cheese, yogurt, cheese, sour creams, cake icing and glazes, whipped topping, leavened and unleavened baked goods, and the like.

In forming food compositions, the dry binder blend of thermal gelling cellulose ether/nanocrystalline cellulose can be typically mixed with foodstuffs during processing. Water in the food stuff dissolves/disperses the binder. The binder can also be added as an aqueous dispersion, if desired. Depending on the product, the binder/food combination may or may not be processed further. Certain products may be simply blended together. Others require further processing such as extruding, heating, cooling, etc.

Thermal gelling cellulose ethers particularly useful in the binder for food compositions are higher molecular weight methyl cellulose and hydroxypropylmethyl cellulose. The binder is typically used in food compositions at levels of about 0.01 to about 5 weight percent solids based upon the total weight of the food composition.

The thermal gelling cellulose ether/nanocrystalline cellulose blend is particularly useful to formulate pharmaceutical capsules. A drug capsule comprises a first portion and a second portion. The first portion interfits the second portion to form a complete capsule. Both portions comprise a blend of thermal gelling cellulose and nanocrystalline cellulose. Capsules formed from the thermal gelling cellulose ether/nanocrystalline cellulose blend in the presently disclosed and claimed inventive concept(s) may exhibit substantially less distortion after drying than capsules formed from conventional cellulose ethers. Particularly thermal gelling cellulose ethers useful in this blend are methyl cellulose and hydroxypropylmethyl cellulose of low molecular weight, i.e., about 3 to about 100 cP. In one non-limiting embodiment, the cellulose ether with about 3 to about 15 cP in a two percent aqueous solution can be used.

As used in this application, the term "capsule" represents a shell for packaging a drug, a vitamin, a nutritional supplement, a cosmeceutical, or a mixture for oral use.

Plasticizers that can be used optionally in forming the capsule include, but are not limited to, glycerine, propylene glycol, polyethylene glycol (PEG 200-6000), diethyl phthalate, dibutyl phthalate, dibutyl sebacate, triethyl citrate, acetyltriethyl citrate, acetyltributyl citrate, tributyl citrate, triacetyl glycerine, castor oil, acetylated monoglyceride, coconut oil, and the like. Optional additives such as colorants and pigments can be included in the capsule as desired.

Thermal gelling cellulose ether capsules can be manufactured by dipping hot pins in a cold, aqueous thermal gelling cellulose ether/nanocrystalline cellulose coating dispersion or by dipping cold pins in a hot, aqueous cellulose ether/nanocrystalline cellulose coating dispersion. The dispersions gel on the pins and water evaporates during a drying step to form thin film layers of dried thermal gelling cellulose ether/nanocrystalline cellulose around the pins. The thin films take the form of caps and bodies, which can be removed from the pins and mated to form capsules. The coating dispersion can include about 15 to about 30% by weight of the combined thermal gelling cellulose ether and nanocrystalline cellulose. Processes for making capsules are disclosed in U.S. Pat. Nos. 3,617,588; 4,001,211; 4,917,885; and 5, 756,036.

While the above disclosure details drug capsules using a blend of thermal gelling cellulose ether and nanocrystalline cellulose, the technology finds broad applications in various commercial areas. For example, but not by way of limitation, the capsules can be formed to accommodate sizes for controlled-or sustained-release applications in industrial, agricultural, or household use. For example but not by way of limitation, the capsules can be sized and manufactured for any application in which sustained-release is desired and can include any compounds and compositions that are chemically compatible with cellulose such as, for example, fragrances, perfumes, fertilizing agents, poisons, herbicides, pesticides, fungicides, antibacterial agents, industrial or household cleaning agents, cleansing agents, personal grooming compositions, or the like.

The blend of cellulose ether and nanocrystalline cellulose of the presently disclosed and claimed inventive concept(s) can further be used as a binder in forming ceramic structures. Generally, in this process precursor ceramic compounds or powders can be blended with a dry thermal gelling cellulose ether/nanocrystalline cellulose binder to form a dry precursor blend. This dry precursor blend is then mixed with minimum liquid vehicle to form a batch which is subjected to a forming process to establish a shape which is dried to form a green ceramic body. The green ceramic body is then sintered at high temperature to create the ceramic product. The liquid vehicle used can include, but are not limited to, water, alcohols, glycerin, organic solvents, or a mixture thereof.

The precursor ceramic compounds are well known and can include compounds such as clay, talc, silica, alumina, magnesia and zirconia, and silicates such as kaolinite, aluminum silicate, silicon carbide, silicon nitride, aluminum titanate and cordierite, and combinations thereof. Other precursor compounds are further disclosed in U.S. Pat. 6,207,101, the disclosure of which is hereby incorporated by reference in its entirety.

Generally, the binder of thermal gelling cellulose ether and nanocrystalline cellulose can make up about 2 to about 15 percent by weight of the dry precursor blend. Certain ceramic applications can include up to about 30% binder.

An amount of water effective to disperse a portion, preferably all, of the binder, can be mixed with the dry precursor blend, Generally, about 10 to 35 percent by weight water, and, more likely, about 30%, can be added to the batch.

The batch can include additional components such as stearic acid, oil, dispersants and flocculants. Other potential additives are disclosed in U.S. Pat. 6,207,101.

The batch is fed into an extruder and is degassed via vacuum. The plastified mass is pushed with a screw through a dye to shape the material. This generates heat. The extruded shape is cut as desired and dried using conventional methods such as convection, infrared, microwave, or radio frequency. This forms the green product. The green product is typically heated to a temperature of about 200 to 450 °C, depending upon the precursor powder, either in a nitrogen or air environment. The heat causes the binder to vaporize and migrate from the green product which then sinters forming the ceramic product.

The combination of the cellulose ether and nanocrystalline cellulose of the presently disclosed and claimed inventive concept(s), due to its improved gel strength, can impart improved mechanical strength to the green product before or during drying resulting in fewer defects.

## Claims

1. A green ceramic body comprising: a ceramic precursor powder effective to form a ceramic upon heating; and a binder, wherein said binder comprises a blend of water soluble thermal gelling cellulose ether and nanocrystalline cellulose.

2. The green ceramic body of claim 1 wherein said ceramic precursor powder comprises compounds selected from the group consisting of clay, talc, silica alumina, magnesium oxide, silicon nitride, silicon carbide, aluminum silicate, aluminum titrate, cordierite, and combinations thereof.

3. The green ceramic body of claim 2 wherein said binder is from 2 to 15 weight percent.

4. The green ceramic body of claim 1 wherein said thermal gelling cellulose ether is selected from the group consisting of methyl cellulose, methylhydroxypropyl cellulose, methylhydroxyethyl cellulose, and combinations thereof.

5. The green ceramic body of claim 1 wherein a ratio of said cellulose ether to said nanocrystalline cellulose is from 10:1 to 1:2 by weight.

6. The green ceramic body of claim 1 further comprising an amount of water effective to dissolve at least a portion of said binder.

7. A method of forming a green ceramic body comprising: blending a ceramic precursor powder with a binder and water to form a blend; and extruding said blend under pressure to form the green ceramic body, wherein said binder comprises water soluble thermal gelling cellulose ether, and nanocrystalline cellulose.

8. The method of claim 7 further comprising adding at least one of a lubricant, a dispersant and a f!occulant into said blend.

9. The method of claim 7 wherein said thermal gelling cellulose ether is selected from the group consisting of methyl cellulose, methylhydroxypropyl cellulose, methylhydroxyethyl cellulose, and combinations thereof.

10. A food product comprising: a food stuff; and a binder, wherein said binder comprises a blend of water soluble thermal gelling cellulose ether and nanocrystalline cellulose.

11. The food product of claim 10 wherein said food stuff is selected from the group consisting of vegetable, meat, soy, seafood, cheese, cream, and combinations thereof.

12. The food product of claim 10 wherein said binder comprises from 0.1 to 5 weight percent.

13. A drug capsule comprising: a first portion; and a second portion, wherein said first portion interfits with said second portion to form a complete capsule, and said first portion and said second portion both comprise a blend of thermal gelling cellulose ether and nanocrystalline cellulose.

14. The drug capsule of claim 13 wherein said thermal gelling cellulose ether is selected from the group consisting of methyl cellulose, methylhydroxypropyl cellulose, methylhydroxyethyl cellulose, and combinations thereof.

15. A method of forming a drug capsule comprising: dipping a pin into an aqueous dispersion of thermal gelling cellulose ether and nanocrystalline cellulose to form a film on said pin; and removing said pin from said dispersion, wherein said film solidifies to form a half of a capsule.

## Patentansprüche

1. Keramikgrünkörper, umfassend: ein Keramikvorläuferpulver, das wirksam ist, um beim Erwärmen eine Keramik zu bilden; und ein Bindemittel, wobei das Bindemittel ein Gemisch aus wasserlöslichem, thermisch gelierendem Celluloseether und nanokristalliner Cellulose umfasst.

2. Keramikgrünkörper nach Anspruch 1, wobei das Keramikvorläuferpulver Verbindungen ausgewählt aus der Gruppe bestehend aus Ton, Talkum, Siliciumdioxid-Aluminiumoxid, Magnesiumoxid, Siliciumnitrid, Siliciumcarbid, Aluminiumsilikat, Aluminiumtitrat, Cordierit und Kombinationen davon umfasst.

3. Keramikgrünkörper nach Anspruch 2, wobei das Bindemittel 2 bis 15 Gew.% beträgt.

4. Keramikgrünkörper nach Anspruch 1, wobei der thermisch gelierende Celluloseether ausgewählt ist aus der Gruppe bestehend aus Methylcellulose, Methylhydroxypropylcellulose, Methylhydroxyethylcellulose und Kombinationen davon.

5. Keramikgrünkörper nach Anspruch 1, wobei ein Verhältnis des Celluloseethers zu der nanokristallinen Cellulose 10:1 bis 1:2 beträgt, bezogen auf das Gewicht.

6. Keramikgrünkörper nach Anspruch 1, ferner umfassend eine Menge an Wasser, die wirksam ist, um mindestens einen Anteil des Bindemittels aufzulösen.

7. Verfahren zum Bilden eines Keramikgrünkörpers, umfassend: Mischen eines Keramikvorläufers mit einem Bindemittel und Wasser, um ein Gemisch zu bilden; und Extrudieren des Gemisches unter Druck, um den Keramikgrünkörper zu bilden, wobei das Bindemittel wasserlöslichen, thermisch gelierenden Celluloseether und nanokristalline Cellulose umfasst.

8. Verfahren nach Anspruch 7, ferner umfassend Zugeben von mindestens einem von einem Schmiermittel, einem Dispergiermittel und einem Flockungsmittel zu dem Gemisch.

9. Verfahren nach Anspruch 7, wobei der thermisch gelierende Celluloseether ausgewählt ist aus der Gruppe bestehend aus Methylcellulose, Methylhydroxypropylcellulose, Methylhydroxyethylcellulose und Kombinationen davon.

10. Nahrungsmittelprodukt, umfassend: ein Nahrungsmittelmaterial und ein Bindemittel, wobei das Bindemittel ein Gemisch aus wasserlöslichem, thermisch gelierendem Celluloseether und nanokristalliner Cellulose umfasst.

11. Nahrungsmittelprodukt nach Anspruch 10, wobei das Nahrungsmittelmaterial ausgewählt ist aus der Gruppe bestehend aus Gemüse, Fleisch, Soja, Meeresfrüchten, Käse, Creme/Sahne/Rahm und Kombinationen davon.

12. Nahrungsmittelprodukt nach Anspruch 10, wobei das Bindemittel 0,1 bis 5 Gew.% umfasst.

13. Arzneikapsel, umfassend: einen ersten Anteil und einen zweiten Anteil, wobei der erste Anteil und der zweite Anteil zusammenpassen, um eine vollständige Kapsel zu bilden, und wobei sowohl der erste Anteil als auch der zweite Anteil ein Gemisch aus thermisch gelierendem Celluloseether und nanokristalliner Cellulose umfassen.

14. Arzneikapsel nach Anspruch 13, wobei der thermisch gelierende Celluloseether ausgewählt ist aus der Gruppe bestehend aus Methylcellulose, Methylhydroxypropylcellulose, Methylhydroxyethylcellulose und Kombinationen davon.

15. Verfahren zum Bilden einer Arzneikapsel, umfassend: Tauchen eines Dorns in eine wässrige Dispersion von thermisch gelierendem Celluloseether und nanokristalliner Cellulose, um einen Film auf dem Dorn zu bilden; und Entfernen des Dorns aus der Dispersion, wobei der Film erstarrt, um eine Hälfte einer Kapsel zu bilden.

## Revendications

1. Corps céramique vert comprenant : un précurseur de céramique en poudre pouvant former efficacement une céramique lors de son chauffage ; et un agent liant, où ledit agent liant comprend un mélange d'éther de cellulose thermogélifiant hydrosoluble et de la cellulose nanocristalline.

2. Corps céramique vert selon la revendication 1, où ledit précurseur de céramique en poudre comprend des composés choisis dans le groupe constitué par argile, talc, silice alumine, oxyde de magnésium, nitrure de silicium, carbure de silicium, silicate d'aluminium, titrate d'aluminium, cordiérite et leurs combinaisons.

3. Corps céramique vert selon la revendication 2, où ledit agent liant est compris entre 2 et 15 pour cent en masse.

4. Corps céramique vert selon la revendication 1, où ledit éther de cellulose thermogélifiant est choisi dans le groupe constitué par méthylcellulose, méthylhydroxypropylcellulose, méthylhydroxyéthylcellulose, et leurs combinaisons.

5. Corps céramique vert selon la revendication 1, où un rapport dudit éther de cellulose sur ladite cellulose nanocristalline est compris entre 10:1 et 1:2 en masse.

6. Corps céramique vert selon la revendication 1 comprenant en outre une quantité d'eau permettant de dissoudre efficacement au moins une partie dudit agent liant.

7. Procédé de formation d'un corps céramique vert comprenant : le mélangeage d'un précurseur de céramique en poudre avec un agent liant et de l'eau pour former un mélange ; et l'extrusion dudit mélange sous pression pour former le corps céramique vert, où ledit agent liant comprend de l'éther de cellulose thermogélifiant hydrosoluble et de la cellulose nanocristalline.

8. Procédé selon la revendication 7, comprenant en outre l'ajout d'au moins l'un des membres du groupe constitué par un lubrifiant, un agent dispersant et un floculant dans ledit mélange.

9. Procédé selon la revendication 7, où ledit éther de cellulose thermogélifiant est choisi dans le groupe constitué par méthylcellulose, méthylhydroxypropylcellulose, méthylhydroxyéthylcellulose, et leurs combinaisons.

10. Produit alimentaire comprenant : une denrée alimentaire ; et un agent liant, où ledit agent liant comprend un mélange d'éther de cellulose thermogélifiant hydrosoluble et de cellulose nanocristalline.

11. Produit alimentaire selon la revendication 10, où ladite denrée alimentaire est choisie dans le groupe constitué par les légumes, la viande, le soja, le poisson et les fruits de mer, le fromage, la crème et leurs combinaisons.

12. Produit alimentaire selon la revendication 10, où ledit agent liant constitue entre 0,1 et 5 pour cent en masse.

13. Capsule de médicament comprenant : une première portion ; et une deuxième portion, où ladite première portion s'imbrique dans ladite deuxième portion pour former une capsule complète, et ladite première portion et ladite deuxième portion comprennent toutes deux un mélange d'éther de cellulose thermogélifiant et de cellulose nanocristalline.

14. Capsule selon la revendication 13, où ledit éther de cellulose thermogélifiant est choisi dans le groupe constitué par méthylcellulose, méthylhydroxypropylcellulose, méthylhydroxyéthylcellulose, et leurs combinaisons.

15. Procédé de formation d'une capsule de médicament comprenant : le trempage d'une aiguille dans une dispersion aqueuse d'éther de cellulose thermogélifiant et de cellulose nanocristalline pour former une pellicule sur ladite aiguille ; et le retrait de ladite aiguille de ladite dispersion, où ladite pellicule se solidifie pour former une moitié de capsule.
